# FASCICULE DE BREVET EUROPEEN

(11) **EP 2 317 973 B1**
(45) Date de publication et mention de la délivrance du brevet: **25.03.2015**
(21) Numéro de dépôt: 09784294.2
(22) Date de dépôt: 23.07.2009
(51) Int. Cl.: A23K 1/18, A61K 9/28, A61K 9/48, A61K 31/197, A61K 31/4164, A23K 1/00, A61K 9/00

(54) **MEDICAMENT APPETISSANT A ADMINISTRATION ORALE SOUS FORME SOLIDE**
APPETITANREGENDES MEDIKAMENT ZUR ORALEN VERABREICHUNG IN FESTER FORM
APPETISING MEDICAMENT FOR ORAL ADMINISTRATION IN SOLID FORM

(30) Priorité: 23.07.2008 FR 0804184
(43) Date de publication de la demande: 11.05.2011
(73) Titulaire: VIRBAC, 06516 Carros (FR)
(72) Inventeur: DERRIEU, Guy, F-06800 Cagnes Sur Mer (FR); CORVAISIER, David, F-06560 Valbonne (FR)
(74) Mandataire: Bernstein, Claire Jacqueline
(86) Numéro de dépôt international: PCT/FR2009/000916
(87) Numéro de publication internationale: WO 2010/010256

(56) Documents cités:
- EP-A- 0 025 226
- EP-A- 0 320 320
- WO-A-89/12442
- FR-A- 2 350 105
- US-A- 5 683 722

## Description

La présente invention se rapporte au domaine de la préparation de médicaments vétérinaires et vise plus particulièrement à améliorer la prise de médicaments, formulés sous une forme solide, par les animaux (domestiques, d'élevage ou sauvages).

On assiste actuellement à une évolution des soins qui sont prodigués aux animaux de compagnie, et la voie orale devient une voie privilégiée pour l'administration de médicaments par le professionnel de santé ou par le propriétaire. En effet, les voies d'administration parentérales usuelles (intramusculaire, sous-cutanée, intradermique ou intraveineuse) des médicaments présentent certains inconvénients. Les voies intramusculaire ou sous-cutanée peuvent, par exemple, être à l'origine d'hématomes ou d'abcès. Quant à la voie intraveineuse, elle requiert souvent l'intervention d'un spécialiste (vétérinaire). Ces voies d'administration parentérales nécessitent également la contention des animaux. Par ailleurs, certains principes actifs sont difficiles à formuler dans des formes galéniques parentérales. Enfin, certains principes actifs n'exerceront leur action thérapeutique chez l'animal que s'ils arrivent directement dans l'appareil digestif.

Les formulations galéniques adaptées à l'administration de médicaments par voie orale ou *per os* se présentent généralement sous forme liquide (tels que des sirops, des solutions ou des suspensions buvables, des gouttes...), sous forme semi-solide (telle que les pâtes pour administration orale) ou sous forme solide. Les formes solides couramment utilisées pour administrer des médicaments aux animaux de compagnie, en particulier les chats et les chiens, se présentent sous diverses formulations de nature différente et sont obtenues par différents procédés. On distingue par exemple les comprimés, les cachets, les capsules, les gélules, les gommes à mâcher, les pilules, les pastilles, les tablettes. On constate que l'observance des traitements administrés par voie orale (c'est-à-dire le respect des consignes et directives du professionnel de santé concernant la prise des médicaments) n'est pas toujours convenablement suivie, en raison de la difficulté à administrer en totalité les traitements aux animaux. En effet, l'administration aux animaux de médicaments sous forme galénique solide par voie orale est souvent difficile du fait du mauvais goût de certaines substances actives ou de certains excipients constituant le médicament et du sens très développé de l'odorat et du goût des animaux. De plus, l'administration orale d'un médicament implique, dans la majorité des cas, l'utilisation de doses variables en fonction des individus et des pathologies considérées et nécessite parfois d'être fractionnée et répétée dans le temps. On a observé, particulièrement chez les chiens et chats, que la principale raison qui rend très difficile, voire impossible, l'observance d'un traitement oral est le défaut d'appétence suscitée par le médicament.

L'appétence pour un médicament se définit comme l'état psychologique correspondant à un désir d'absorber le médicament en réponse à la perception des caractères organoleptiques de celui-ci. La capacité à susciter l'appétence est appelée appétissance. Elle correspond à la combinaison des caractères d'un médicament qui déterminent l'attrait qu'il aura sur des animaux normalement nourris. L'appétissance d'un médicament participe grandement au refus ou à l'acceptation par l'animal de la prise spontanée du traitement et de la répétition de la prise sur des périodes parfois longues. Dans le cadre de certains traitements, la prise du traitement peut être journalier et à vie.

L'appétissance d'un médicament administré par voie orale conduit à l'acceptation et à l'ingestion volontaire par les animaux. Cette appétissance peut être mesurée dans un essai général d'appétence prenant en compte différents paramètres du médicament, comme sa prise spontanée à la main ou au sol, ou encore sa consommation, même s'il y a plusieurs médicaments à prendre en une prise ou à intervalles réguliers par l'animal.

Dans l'art antérieur, de nombreuses solutions ont été mises au point pour faciliter l'absorption des médicaments par l'animal :
1. Une première option consiste à masquer le goût et/ou l'odeur désagréable du ou des principes actifs par encapsulation ou par enrobage de ces derniers.

Ainsi, dans la Demande de Brevet EP 0 997 143, le problème d'amertume des principes actifs est résolu en les encapsulant dans des compositions qui masquent le goût, telles que des mélanges d'acétate de cellulose ou d'acétate butyrate de cellulose et de polyvinylpyrrolidone (PVP) ou d'hydroxypropyl cellulose (HPC).

La Demande de Brevet EP 1 490 037 propose la préparation d'un médicament dont le goût désagréable du principe actif est masqué ; la préparation consiste à enrober le principe actif dont on cherche à masquer le goût autour de granules servant de support ; la couche de principe actif est ensuite recouverte d'une couche protectrice réalisée avec une matrice polymérique physiologiquement acceptable (telle que cellulose, amidon, saccharose, lactose ou d'autres types de sucre) qui évite au principe actif d'être directement en contact avec les cellules gustatives de l'animal. Les granules ainsi préparés peuvent ensuite être mélangés avec une substance appétissante, le mélange est alors comprimé sous forme de tablette par exemple.

Cependant, ces solutions nécessitent de nombreuses étapes d'encapsulation ou d'enrobage. De plus, la préparation des produits médicamenteux sous forme galénique solide se fait ensuite par compression ; or, certains principes actifs sont très fragiles et ne résistent pas aux contraintes physiques utilisées lors de la compression.

La Demande Internationale WO 01/15547 décrit une formulation d'agents actifs mal acceptés par l'animal pour l'utilisation vétérinaire. Là encore, le principe proposé est d'encapsuler le ou les agents actifs, préalablement incorporés dans une matrice, pour en masquer le goût. La matrice intégrant les agents actifs est recouverte d'une couche ayant un goût neutre comprenant du xanthane, du polyvinylpyrrolidone et du lauryl-sulfate de sodium. Ainsi formulé(s), le ou les agents actifs ne sont plus perçu(s) par l'animal.

Cependant, le procédé décrit dans ce document nécessite notamment une étape d'extrusion qui est susceptible de dégrader les principes actifs fragiles.
2. Selon une autre option, les principes actifs sont mélangés dans une matrice comprenant une substance appétissante afin de masquer leur goût.

Ainsi, la Demande de Brevet australien AU2001279664 décrit un produit extrudé à base d'amidon contenant un principe actif et un arôme spécifique, apte à être administré oralement à un animal.

Cependant, l'extrusion peut poser des problèmes de dégradation de certains principes actifs pour lesquels il est préférable de l'éviter.

Par ailleurs, un arôme est un principe odorant d'origine synthétique ou naturelle qui ne sera perçu que par l'odorat. En particulier, il ne produira pas de sensation sur l'organe du goût et n'aura donc pas de saveur. Aussi, la qualité d'un arôme en tant que matière susceptible d'augmenter l'appétissance est limitée.

La Demande de Brevet EP 0 320 320 décrit un comprimé pour animal domestique caractérisé en ce qu'il est constitué par au moins un noyau contenant un ou plusieurs principes actifs totalement englobés dans une matrice appétante pour l'animal. L'appétissance du comprimé est conférée par des agents tels que la poudre de foie ou la levure de bière qui sont des constituants de la matrice qui contient, par ailleurs, des excipients de compression et/ou des agents lubrifiants dont le rôle est de faciliter la fabrication du comprimé.

Cependant, la préparation de ces comprimés demande une étape de compression qui constitue un inconvénient en ce qu'elle est susceptible d'endommager le ou les principes actifs. Par ailleurs, le ou les principes actifs se retrouvent être en contact intime avec les constituants appétants de la matrice qui ne sont pas nécessairement compatibles pour assurer une bonne stabilité aussi bien aux principes actifs qu'aux autres constituants de la matrice, notamment les constituants appétants de la matrice.

La Demande de Brevet EP 0 725 570 de la demanderesse décrit une composition attractive pour l'animal sous forme solide, comprenant de 3 à 20% d'un polymère insoluble dans l'eau, de 5 à 45% en poids d'une substance appétissante et de 35 à 60% en poids de substance lipidique obtenue par fusion des substances lipidiques sous forme solide à une température inférieure à celle du point de fusion du/des polymères et mélange du/des polymères aux autres composants à la même température. La composition peut comprendre jusqu'à 50% en poids de substance bioactive et être mise sous forme de blocs. Il s'agit d'une formulation présentant une grande résistance mécanique pour des médicaments susceptibles d'être disséminés dans la nature pour traiter, par exemple, des animaux sauvages. La substance appétissante est répartie uniformément dans la forme galénique résultante.

La préparation de telles compositions nécessite un chauffage compris entre 40 et 80°C pour obtenir la fusion de certains constituants. Ce chauffage peut cependant constituer un inconvénient majeur pour la stabilité de certains principes actifs.

Les limites des formulations dans lesquelles les arômes et/ou les matières appétissantes et les principes actifs sont mélangées et donc en contact sont :
- qu'elles peuvent conduire à des incompatibilités entraînant la transformation ou dégradation du ou des principes actifs ou des arômes et des matières appétissantes. De plus, même présentes en quantité importante, les matières appétissantes ne masquent pas toujours l'odeur ou le goût désagréable à l'animal qui a un odorat et un goût très développés ; cela est d'autant plus vrai si la formulation contient des lipides, liquides ou solides, qui sont connus pour exalter les saveurs ;
- que la répartition homogène de la matière appétissante à l'intérieur des compositions rend la majorité de la matière appétissante indisponible pour exercer l'appétissance maximale avant que l'animal n'ait la formulation en bouche et ne commence à la mastiquer pour en ressentir le goût, le cas échéant ; et
- que ces formulations ne permettent pas toujours de protéger le principe actif des conditions environnementales telles que l'humidité et la température et de maintenir une parfaite stabilité dans le temps.

3. Encore une autre option pour faciliter l'administration orale de principes actifs est d'enrober le médicament dans des matières appétissantes.

Les Demandes de Brevet suivantes : FR 2 715 803, US 5,853,757, US 6,143,316, US 5,792,470, US 5,674,515, EP 0 574 301, US 4,857,333, DE 198 53 729, WO 03/030863, WO 2004/043427, WO 2007/090987, proposent des leurres réalisés avec des matières appétissantes dans lesquels on peut introduire extemporanément un médicament sous forme solide afin de l'administrer oralement à l'animal. Ces systèmes présentent l'avantage de s'adapter à de nombreuses formes solides de médicaments vétérinaires pour la voie orale.

Selon un principe similaire, la Demande WO 89/12442 décrit un support de composition vétérinaire pour poisson comprenant une couche externe imperméable à l'eau et aux principes actifs ; cette couche externe enveloppe au moins une chambre contenant au moins un principe actif et masque le goût dudit principe actif. La couche externe est constituée d'un matériau végétal ou animal, de préférence, de la farine de poisson, et/ou d'un extrait aqueux de matériau marin, et éventuellement d'un exhausteur de goût et/ou d'un liant. Une caractéristique importante de ce support de composition vétérinaire pour poisson est son caractère imperméable, notamment à l'eau, qui est obtenu par l'utilisation d'un liant dérivé d'amidon et par la présence de l'extrait aqueux de matériau marin qui agit comme une colle naturelle.

L'inconvénient commun de ces leurres est que leur utilisation nécessite l'opération préalable d'introduction du médicament dans le leurre, ce qui peut rebuter certains utilisateurs et aussi devenir gênant lorsqu'un grand nombre d'animaux doit être traité. De plus, leur volume important (nécessairement plus gros que le médicament) demande une quantité de matière importante et leur fabrication doit être adaptée à leur forme complexe ; ainsi ces leurres s'avèrent souvent coûteux.

La Demande de Brevet EP 0 725 627 de la demanderesse décrit une forme galénique pour l'administration orale de substances bioactives (biologiques, chimiques ou médicamenteuses) du type appât. Il s'agit d'une forme galénique à double compartiment comprenant :
(i) à titre de premier compartiment, un noyau central solide poreux contenant une ou plusieurs substances bioactives ; ce noyau a une composition particulière adaptée à une solidification par lyophilisation et il présente la caractéristique de se dissoudre ou de se désintégrer rapidement dans la salive ; ainsi, lorsque l'animal mâche ce médicament, les fragments générés par le noyau vont adhérer aux tissus de la cavité buccale de l'animal ; et
(ii) un second compartiment qui est une couche externe hydrophobe contenant de 3 à 30% d'une substance appétissante naturelle ou synthétique du type farine de viande ou de poisson, de 40 à 93% d'une substance lipidique et de 4 à 30% de polymère. La composition du second compartiment a comme spécificité de comporter des lipides afin de la rendre hydrophobe et de protéger le noyau contre l'eau, elle comporte également des polymères qui sont une charge structurelle ayant pour rôle de consolider la couche lipidique, de faciliter la manipulation de l'appât et de modifier le point de fusion de la composition ; cette couche est ainsi hydrophobe, appétante et d'épaisseur contrôlée. Cependant, la préparation d'une telle formulation impose des contraintes mécaniques et thermiques défavorables à la stabilité de certains principes actifs.

Dans les présentations "gélules dures" ou "capsules molles", formes usuellement rejetées par l'animal, il est possible d'ajouter des arômes dans la tunique (paroi des gélules ou des capsules). La teneur en arôme est cependant nécessairement faible en raison des constituants de la tunique et de son poids faible par rapport au poids total des gélules ou capsules pleines. Ainsi, la présence d'arôme n'est souvent pas suffisante pour exercer une appétissance souhaitée et favoriser la prise, d'autant plus que l'arôme n'est perçu que par l'odorat, il ne produit pas de sensation sur l'organe du goût et n'a donc pas de saveur. Par ailleurs, la quantité d'arôme, sous forme liquide, introduite ne peut qu'être faible (généralement inférieure à 2 pour cent), et doit être compatible avec la nature du composant de la tunique qui est dans la majorité des cas la gélatine.

La Demande de Brevet EP 0 025 226 décrit des compositions d'enrobage de diverses formes solides alimentaire ou pharmaceutique à usage humain ; lesdites compositions sont constituées de saccharose, d'au moins un autre sucre, tel que le lactose, d'eau et peuvent en outre comprendre des colorants, agents de saveur, parfums et adjuvants. Ces compositions facilitent la mise en oeuvre d'un procédé d'enrobage par dragéification grâce à une composition en sucres qui les rend moins susceptibles de cristalliser en cours de procédé. L'enrobage en sucre, comme dans toutes les dragées, confèrent un goût agréable pour l'homme. Ce type de formulation n'est cependant pas adapté aux animaux, les sucres purifiés n'étant pas une matière appétissant pour les animaux ; en outre, ils ne dégageant pas une odeur nécessaire à provoquer l'appétissance des animaux. Enfin, la forme "dragée" est mal acceptée par les animaux. Cette forme extrêmement dure et de surface très lisse est difficilement préhensible par les animaux.

En outre, une telle composition d'enrobage n'est pas compatible avec un éventuel ajout de matière appétissante pour animaux sous forme pulvérulente car :
- le sirop de sucre fortement chargé en matière appétissante est susceptible de provoquer un mauvais écoulement dans les buses de dragéification au cours du procédé de préparation des dragées ;
- la cristallisation du sucre lors de l'évaporation du solvant lors de la préparation des dragées aura pour effet de piéger la matière appétissante, diminuant ainsi considérablement l'attrait de la matière appétissante pour l'animal.

FR 2350105 décrit des préparations vétérinaires dont l'ingestion par des animaux est facilitée par l'utilisation d'une matière odorante et/ou gustative spécifique de l'animal auquel est destinée la préparation.

Il existe donc un besoin d'améliorer l'appétissance de médicaments solides administrés par voie orale aux animaux.

La demanderesse s'est donnée pour but de pallier les inconvénients de l'Art Antérieur et de mettre au point une composition et un procédé permettant de conférer aux médicaments à administration orale sous forme solide une meilleure appétissance que les compositions et techniques connues. Elle s'est en particulier employée à mettre au point une composition vétérinaire à administration orale simple, économique, utilisable quelle que soit la forme galénique solide mise en jeu et dont la fabrication soit facilement industrialisable. En particulier, le traitement pour améliorer l'appétissance du médicament doit pouvoir être appliqué en fin de chaîne de fabrication dudit médicament, tout en assurant la stabilité chimique et physique de celui-ci.

Dans les travaux qui ont conduit à la mise au point de la composition d'enrobage selon l'invention, la demanderesse a constaté que l'adjonction d'une substance appétissante pour l'animal cible, se présentant sous forme pulvérulente à l'état de matière première, en quantité concentrée à la surface d'une composition solide administrée par voie orale, favorisait d'une part l'appétence de l'animal, et d'autre part incitait l'animal à consommer ladite composition solide. Les substances qui confèrent l'appétissance à une composition solide administrée par voie orale dépendent des goûts et des odeurs perçues et appréciées par l'animal cible.

L'invention se fonde sur cette constatation pour proposer un médicament appétissant très bien accepté par les animaux, absorbé rapidement, qu'il soit donné de façon occasionnelle ou répétée.

La présente invention a en premier lieu pour objet une composition d'enrobage contenant une quantité adéquate d'une matière appétissante apte à être appliquée par un procédé de pelliculage (consistant à appliquer une fine couche de la composition d'enrobage en surface) sur une composition pharmaceutique vétérinaire solide à administration orale.

Ainsi, la présente invention se rapporte à une composition d'enrobage apte à être appliquée par un procédé de pelliculage sur une composition pharmaceutique vétérinaire solide à administration orale caractérisée en ce :
(i) qu'elle comprend :
   - une matière appétissante pour l'animal cible, se présentant sous forme pulvérulente, choisie parmi les substances d'origine animale ou végétale, directement mises en poudre après traitements tels que le séchage ou la déshydratation, le broyage, le calibrage mais aussi après transformation avec ajout d'autres composants pour favoriser la conservation par exemple. Parmi les substances de choix qui ont une forte appétissance pour les espèces cibles, en particulier les carnivores domestiques tels que les chiens et les chats, on trouve de manière non limitative la levure de bière, les farines de viandes, les farines de poissons, les poudres de fromages ou dérivés du lait, la poudre de foie et leur mélange ;
   - au moins un liant choisi parmi les polymères d'alcool polyvinylique, de polyvinylpyrrolidone, les copolymères de vinylpyrrolidone et d'acétate de vinyle, l'acétophthalate de polyvinyle, les celluloses et leurs dérivés, l'acide alginique et ses sels, la zeïne, l'acide hyaluronique, les pectines, la gomme arabique, la gomme adragante, la gomme karaya, la gomme xanthane, les carraghénanes, les polymères pullulan ou agar, le chitosan ou ses dérivés, les carbomères, l'acide acrylique réticulé avec des polyalkényl éthers, les polycarbophiles, les copolymères de méthylvinyl et d'anhydride maléique, les poly-saccharides ou le mélange d'au moins deux de ces liants ; étant entendu que lorsque le liant comprend un ou plusieurs polysaccharides, le pourcentage en poids du ou desdits polysaccharides représente 50% ou moins, de préférence 25% ou moins, du poids total de liants ; et
   - un solvant choisi parmi l'eau, le méthanol, l'éthanol, l'isopropanol, le propylène glycol, la glycérine ou leur mélange ; et
(ii) qu'elle contient entre 30% et 90% inclus en poids de matière appétissante par rapport au poids total du mélange constitué par le liant et la matière appétissante.

La matière appétissante pour l'animal cible se présente avantageusement sous forme pulvérulente, c'est-à-dire qu'elle est constituée de particules de petite taille ; cette forme contribue significativement au maintien du caractère appétissant pour l'animal de la matière appétissante même après l'enrobage par pelliculage de ladite matière sur une composition pharmaceutique vétérinaire solide à administration orale. En effet, la forme pulvérulente présente, par rapport aux formes liquides notamment, l'avantage de favoriser la restitution de particules et donc de maximiser les surfaces d'échanges avec l'extérieur après application de la composition d'enrobage sur la composition pharmaceutique solide vétérinaire à enrober.

L'attractivité pour l'animal de la composition pharmaceutique solide vétérinaire enrobée dépendra plus particulièrement de la charge importante de la composition d'enrobage en matière appétissante, de sa localisation en surface de la composition pharmaceutique solide vétérinaire et d'une surface d'échange la plus importante possible grâce à la présence de particules de petite taille. Le diamètre moyen des particules constituant la matière appétissante pour l'animal cible, se présentant sous forme pulvérulente, sera avantageusement inférieur à 500 µm, préférentiellement inférieur à 400 µm et encore plus préférentiellement compris entre 50 et 100 µm.

On pourra utiliser comme matière appétissante toute matière alimentaire qui provoque un attrait pour un animal cible ; ledit attrait pouvant être évalué par un test d'appétence.

Le solvant est utilisé pour mettre en solution ou en suspension le liant et la matière appétissante pulvérulente ; sa teneur est choisie par l'homme du métier en fonction de la nature dudit liant et de ladite matière appétissante pulvérulente. Le rapport poids de solvant sur le poids total du mélange constitué par le liant et la matière appétissante est préférentiellement supérieur à 2 inclus et inférieure à 6 inclus et encore plus préférentiellement compris entre 2 et 4 inclus.

Le choix du liant utilisé dans la composition d'enrobage doit rendre disponible la matière appétissante afin qu'elle exerce au mieux sa fonction sur l'animal. Il faut donc veiller à limiter le phénomène d'encapsulation de la matière appétissante sous forme pulvérulente dans des cristaux que pourraient former le liant lors du séchage en surface de la composition d'enrobage après son application sur pharmaceutique solide vétérinaire.

Par celluloses et leurs dérivés, on entend notamment la cellulose microcristalline, les éthers ou esters des alkyl cellulose comme la méthyl cellulose, l'éthylcellulose, l'hydroxypropylcellulose, l'hydroxypropylméthylcellulose, l'acétophthalate de cellulose, l'acétate de cellulose.

Les poly-saccharides peuvent être la dextrine.

Lorsque des poly-saccharides sont utilisés en tant que liants, ils le sont en mélange avec au moins un liant d'une autre famille chimique (c'est-à-dire qui n'est ni un mono-, un di- ou un polysaccharide) et le pourcentage en poids des poly-saccharides n'excède pas 50%, préférentiellement 25%, du poids total des liants.

Les polyols peuvent être choisis parmi le sorbitol, le xylitol, l'isomalt, le maltitol, le mannitol, le lactitol ou leur mélange.

De préférence, la matière appétissante est de la poudre de foie, de la levure de bière ou un mélange de poudre de foie et de levure de bière.

De préférence, le liant utilisé est choisi parmi les celluloses ou encore leurs mélanges comprenant de préférence l'hydroxypropylméthylcellulose et la cellulose microcristalline.

De préférence, la composition d'enrobage contient entre 40% et 90% inclus en poids de matière appétissante par rapport au poids total du mélange constitué par le liant et la matière appétissante, plus préférentiellement elle contient entre 50% et 90%, encore plus préférentiellement entre 60% et 90% inclus en poids de matière appétissante par rapport au poids total du mélange constitué par le liant et la matière appétissante. De manière encore plus préférentielle, la composition d'enrobage contient entre 60% et 80% inclus en poids de matière appétissante par rapport au poids total du mélange constitué par le liant et la matière appétissante.

Ainsi composée de ces trois ingrédients : matière appétissante, liant et solvant, la composition d'enrobage peut être utilisée à l'exclusion de tout autre ingrédient.

Selon une autre variante de l'invention, la composition d'enrobage comprend en outre un ou des additifs acceptables choisis, à titre d'exemple et sans aucun caractère limitatif, parmi les plastifiants comme l'acide stéarique et ses dérivés, l'acide citrique et ses dérivés, l'acide lactique et ses dérivés, le propylène glycols, la glycérine, les phthalates et leurs dérivés, les adipates et leurs dérivés, les sébaçates et leurs dérivés, les polyéthylène glycols et leurs dérivés, les sucres comme le glucose, les maltodextrines, le sorbitol, le saccharose, les monoglycérides acétylés ; des stabilisants comme les antioxydants ; des conservateurs tels que l'acide ascorbique et ses dérivés ou sels, le butylhydroxyanisol, le butylhydroxytoluène, l'acide gallique et ses dérivés, le métabisulfite de sodium ou potassium, le bisulfite de sodium, les vitamines et leurs dérivés, l'EDTA et ses sels, les parabens ; des charges tels que le talc, la silice, les silicates, la cellulose microcristalline, le mica, les carbonates, les silicones ; des surfactants ; des colorants tels que les oxydes de fer, les colorants solubles absorbés sur laques d'alumine, l'oxyde de titane ; ou encore des porogènes.

La présente invention a également pour objet l'utilisation de ladite composition d'enrobage pour l'enrobage par pelliculage d'une composition pharmaceutique vétérinaire solide à administration orale.

La composition pharmaceutique vétérinaire solide à administration orale peut être un médicament pour usage vétérinaire, c'est-à-dire destiné à la prévention et/ou au traitement d'un état pathologique d'un animal, ou bien un produit nutraceutique ou un complément nutritionnel destiné aux animaux de compagnies, en particulier à un animal carnivore domestique tel qu'un chien ou un chat.

L'invention se rapporte également à un procédé d'enrobage par pelliculage qui permet de fixer en surface d'une composition pharmaceutique vétérinaire solide à administration orale une matière appétissante, préférentiellement une matière appétissante pulvérulente. Le pelliculage de la composition pharmaceutique vétérinaire solide à administration orale est obtenu selon les techniques et les matériels classiques connus de l'homme de métier. Les exemples suivants, non limitatifs, illustrent l'invention.

Plus particulièrement, la présente invention se rapporte à un procédé de préparation d'une composition pharmaceutique vétérinaire solide à administration orale enrobée d'une matière appétissante (ci-après désignée composition appétissante pharmaceutique vétérinaire solide à administration orale), caractérisé en ce qu'il comprend les étapes suivantes :
(1) application sur une composition pharmaceutique vétérinaire solide à administration orale d'une couche d'une composition d'enrobage comprenant au moins une matière appétissante pour l'animal cible sous forme pulvérulente, un liant et un solvant ;
(2) séchage de ladite composition enrobée obtenue à l'étape (1) ;
(3) optionnellement, répétition des étapes (1) et (2) ; et
(4) obtention d'une composition appétissante pharmaceutique vétérinaire solide à administration orale dans laquelle ladite matière appétissante représente au moins 5% et de préférence au moins 15% en poids de ladite composition appétissante pharmaceutique vétérinaire solide à administration orale.

Selon un mode de réalisation particulier, le procédé selon l'invention est caractérisé en ce qu'il comprend les étapes suivantes :
(A) introduction d'une composition pharmaceutique vétérinaire solide à administration orale à enrober dans une turbine à dragéifier ;
(B) pulvérisation à l'aide d'une buse d'une composition d'enrobage comprenant au moins une matière appétissante pour l'animal cible sous forme pulvérulente, un liant et un solvant, dans ladite turbine en rotation en une quantité telle que ladite matière appétissante représente au moins 5% et de préférence au moins 15% en poids de la composition appétissante pharmaceutique vétérinaire solide à administration orale.

Les conditions de température pour la mise en oeuvre du procédé sont propres à l'équipement et aux ingrédients utilisés

De façon avantageuse, la mise en oeuvre du procédé selon l'invention est telle que la température de la composition pharmaceutique vétérinaire solide à administration orale ne dépasse pas 42°C, de préférence 40°C et encore préférentiellement 38°C.

L'enrobage nécessite l'utilisation d'une composition d'enrobage comme définie ci-avant, qui est une solution ou une suspension aqueuse ou organique d'au moins un liant choisi à titre d'exemple et sans caractère limitatif parmi, les polymères d'alcool polyvinylique, de polyvinylpyrrolidone, les copolymères de vinylpyrrolidone et d'acétate de vinyle, l'acétophthalate de polyvinyle, les celluloses et leurs dérivés telles que les éthers ou esters des alkyl cellulose comme la méthylcellulose, l'éthylcellulose, l'hydroxypropylcellulose l'hydroxypropylméthylcellulose, l'acétophthalate de cellulose, l'acétate de cellulose, l'acide alginique et ses sels, la zéïne, l'acide hyaluronique, les pectines, la gomme arabique, la gomme adragante, la gomme karaya, la gomme xanthane, les carraghénanes, les polymères pullulan ou agar, le chitosan ou ses dérivés, les carbomères, l'acide acrylique réticulé avec des polyalkenyl éthers, les polycarbophiles, les copolymères de méthylvinyl et d'anhydride maléique, les poly-saccharides choisis parmi les dextrines ; dans un solvant et contenant la matière appétissante.

Lorsque des poly-saccharides sont utilisés en tant que liants, ils le sont en mélange avec au moins un liant d'une autre famille chimique (c'est-à-dire qui n'est ni un mono-, un di- ou un polysaccharide) et le pourcentage en poids des poly-saccharides n'excède pas 50%, préférentiellement 25%, du poids total des liants.

L'enrobage s'obtient par application de la solution ou suspension et évaporation du solvant. Cette application peut être répétée plusieurs fois de manière à obtenir le taux de matière appétissante voulu.

La composition d'enrobage selon l'invention est soit aqueuse soit organique avec des solvants tels que le méthanol, l'éthanol, l'isopropanol, le propylène glycol, la glycérine ou encore une solution hydroalcoolique.

De façon préférée, ladite composition pharmaceutique vétérinaire solide à administration orale comprend au moins un composé actif (indifféremment appelé principe actif ou composé actif par la suite) présentant un goût ou une odeur désagréable qui gêne ainsi la prise de la composition vétérinaire par l'animal cible et/ou un composé actif présentant une instabilité, par exemple vis-à-vis de la matière appétissante, qui gêne l'obtention d'un médicament appétissant ainsi que la prise de la composition vétérinaire par l'animal cible.

Selon une variante de l'objet de l'invention, ladite composition pharmaceutique vétérinaire solide à administration orale comprend au moins un excipient présentant un goût ou une odeur désagréable qui gêne ainsi la prise de la composition vétérinaire par l'animal cible et/ou un excipient présentant une instabilité, par exemple vis-à-vis de la matière appétissante, qui gêne l'obtention d'un médicament appétissant ainsi que la prise de la composition vétérinaire par l'animal cible.

Par excipient, on entend tout ingrédient de ladite composition pharmaceutique vétérinaire solide à administration orale à l'exclusion du ou des principes actifs. Il s'agit, par exemple et sans caractère limitatif, de charges, de conservateurs, des stabilisants, des colorants, des porogènes, des agents de désagrégation.

La composition pharmaceutique vétérinaire solide à administration orale est, par exemple et sans que cette liste ait un caractère limitatif, un comprimé, une gélule, une capsule dure ou molle, une pilule, une tablette, des granulés, un cachet, une gomme à mâcher, une pastille...

En outre, la composition pharmaceutique vétérinaire solide à administration orale est optionnellement sécable. En effet, on s'est rendu compte que le fait de réaliser sur l'une ou les deux faces principales de la composition appétissante pharmaceutique vétérinaire solide à administration orale, une ou plusieurs, notamment deux, entailles faisant office de barre de sécabilité n'enlève rien à la facilité de la prise de ladite composition appétissante pharmaceutique vétérinaire solide à administration orale ou des morceaux obtenus après rupture suivant la ou les barres de sécabilité.

Ainsi, une variante de mise en oeuvre du procédé selon l'invention, consiste à utiliser une composition pharmaceutique vétérinaire solide à administration orale qui a une forme sécable, ce qui lui permet, après l'étape d'enrobage par pelliculage de pouvoir être divisée selon les besoins de l'animal à soigner. L'exemple 6 montre que même après avoir coupé un comprimé sécable enrobé selon le procédé de l'invention, le fragment de la composition appétissante pharmaceutique vétérinaire solide à administration orale est toujours très bien accepté par l'animal.

Selon un autre mode avantageux de mise en oeuvre du procédé selon l'invention, la composition pharmaceutique vétérinaire solide à administration orale est séparée de la composition d'enrobage contenant la matière appétissante par un film qualifié de film primaire. Ce film primaire peut être obtenu par une encapsulation, un enrobage ou un pelliculage de ladite composition pharmaceutique vétérinaire solide à administration orale par un film, principalement polymérique, préalablement à l'enrobage par la composition d'enrobage contenant la matière appétissante. Ce film primaire peut avoir pour fonction de produire, en fonction des constituants choisis, une barrière isolante et protéger ainsi la composition pharmaceutique vétérinaire solide à administration orale, et en particulier le ou les principes actifs, de toutes les attaques physiques ou chimiques susceptibles d'être induites par la composition d'enrobage selon l'invention. Le film primaire peut aussi avoir pour fonction de faciliter l'adhésion de la composition d'enrobage amenant la matière appétissante à la surface de la composition pharmaceutique vétérinaire solide à administration orale, Enfin, le film primaire peut avoir pour fonction à la fois de fournir une barrière isolante à la surface de la composition pharmaceutique vétérinaire solide à administration orale et de faciliter l'adhésion de la composition d'enrobage amenant la matière appétissante à la surface de la même composition pharmaceutique vétérinaire solide à administration orale.

Ainsi, le procédé selon l'invention peut comprendre une étape additionnelle préalable à l'étape (1) et qui consiste à recouvrir, c'est-à-dire à pelliculer ou enrober ladite composition pharmaceutique vétérinaire solide à administration orale d'un film primaire à l'aide d'une solution ou suspension comprenant au moins un liant et au moins un solvant et/ou un ou des additifs acceptables pour l'effet recherché ; le liant, le solvant et les additifs acceptables sont tels que définis précédemment.

Tout comme pour l'application de la composition d'enrobage selon l'invention en surface de la composition pharmaceutique vétérinaire solide à administration orale, l'application préalable du film primaire en surface de la composition pharmaceutique vétérinaire solide à administration orale est réalisée par des techniques classiques d'enrobage ou de pelliculage de compositions pharmaceutiques solides.

La présente invention a également pour objet une composition appétissante pharmaceutique vétérinaire solide à administration orale contenant au moins un principe actif, caractérisée en ce qu'elle est susceptible d'être obtenue par le procédé d'enrobage par pelliculage selon l'invention.

Cette composition appétissante pharmaceutique vétérinaire solide à administration orale est caractérisée en ce qu'elle comprend :
- une composition pharmaceutique vétérinaire solide à administration orale contenant au moins un principe actif ; et
- un enrobage positionné autour de ladite composition constitué d'une matière appétissante et d'un liant ; ledit enrobage contenant entre 30% et 90% inclus en poids de matière appétissante par rapport au poids total de l'enrobage constitué de la matière appétissante et du liant ; ladite matière appétissante représentant au moins 5% et préférentiellement au moins 15% en poids de ladite composition appétissante pharmaceutique vétérinaire solide à administration orale.

La matière appétissante et le liant sont tels que définis ci-dessus.

L'avantage que présente cette forme particulière de composition appétissante pharmaceutique vétérinaire solide à administration orale est que la matière appétissante est concentrée en surface de ladite composition appétissante pharmaceutique vétérinaire solide à administration orale. Ainsi en utilisant une quantité limitée et économique de matière appétissante, on obtient néanmoins une très bonne efficacité de prise du médicament appétissant comme cela est démontré dans les exemples 2, 4, 6 et 8 qui suivent.

Cette composition appétissante pharmaceutique vétérinaire solide à administration orale trouve un intérêt particulier pour l'administration de principes actifs mal acceptés par les animaux. Elle peut cependant être utilisée pour favoriser l'administration de tout actif d'intérêt vétérinaire dans la mesure où elle facilite la prise de la composition vétérinaire et elle favorise la conservation de l'actif.

D'une manière générale, la composition appétissante pharmaceutique vétérinaire solide à administration orale contient entre 0,01 et 50% inclus en poids par rapport au poids total de la composition appétissante pharmaceutique vétérinaire solide à administration orale, d'au moins un principe actif.

D'une manière préférée, la teneur en principe actif ne dépasse pas 35% en poids du poids de la composition appétissante pharmaceutique vétérinaire solide à administration orale.

Bien entendu, la composition appétissante pharmaceutique vétérinaire solide à administration orale conforme à l'invention peut renfermer différents types de principes actifs choisis dans toutes les classes thérapeutiques existantes, parmi lesquelles on peut mentionner sans caractère limitatif les exemples suivants :
- les anti-infectieux tels que les antibiotiques, les sulfamides... ;
- les anti- parasitaires internes ;
- les IGR (Inhibiteurs de croissance des insectes) ;
- les anti-inflammatoires stéroïdiens ou non, et les antihistaminiques ;
- les vaccins oraux ;
- les hormones, telles que les prostaglandines ;
- les substances de thérapie digestive, tels les pansements et sédatifs gastro-intestinaux, les anti-ulcéreux, les flores de substitution, les anti-diarrhéiques, les hépato-protecteurs, les anti-spasmodiques, les laxatifs, les antiseptiques intestinaux ;
- les substances de thérapie respiratoire tels les analeptiques respiratoires, les anti-tussifs, les broncho-dilatateurs, les fluidifiants bronchiques et mucolytiques, les antiseptiques respiratoires ;
- les substances agissant sur le système nerveux tels les analgésiques, les sédatifs et les tranquillisants, les anti-épileptiques, les anesthésiques, les orexigènes et anorexigènes ;
- les substances de thérapie immunitaires telles les interleukines (interféron etc...) ;
- les substances de thérapie anti-cancéreuses tels les antimitotiques, les cytostatiques ;
- les macro, micro et oligo-éléments ;
- les vitamines ;
- les acides aminés et les protéines ;
- les acides gras ;
- les glucides ;
- les extraits de plantes ou d'organes d'animaux (phytothérapie, organothérapie).

Le ou les principes actifs peuvent être simplement répartis au sein de la composition pharmaceutique vétérinaire solide à administration orale ou regroupés dans un noyau lui-même incorporé dans ladite composition pharmaceutique vétérinaire solide à administration orale formant une masse monobloc.

Les principes actifs peuvent être eux-mêmes encapsulés ou enrobés par les techniques connues de l'homme du métier afin d'améliorer leur stabilité ou d'augmenter le masquage de leur odeur et de leur goût à la perception olfactive ou gustative de l'animal.

Enfin, la composition appétissante pharmaceutique vétérinaire solide à administration orale selon l'invention peut se présenter sous la forme d'un solide sécable.

La composition appétissante pharmaceutique vétérinaire solide à administration orale est préférentiellement destinée à un animal carnivore domestique tel qu'un chien ou un chat.

### EXEMPLE 1 - Préparation de comprimés enrobés selon l'invention

### 1- enrobage avec un film primaire isolant

On prépare la dispersion à pulvériser, pour former un film primaire isolant, suivante :
- eau 366,7 g
- hydroxypropylméthylcellulose / cellulose microcristalline / acide stéarique 50,0 g
pour encapsuler 2000 g de comprimés ronds de 250 mg (de composition : métronidazole / hydroxypropylcellulose / cellulose microcristalline / glycérides de béhénate / povidone / silice colloïdale / dioxyde de silice colloïdale / acide silicique léger).

Les comprimés sont introduits dans la turbine à dragéifier où ils sont dépoussiérés et mis en température.

L'enrobage est effectué pendant 2h avec un débit croissant de 3,1 à 4,56 g/min à l'aide d'une buse de 1 mm.

La température de l'air entrant est maintenue à environ 50°C, en vérifiant que la température des comprimés ne dépasse pas 40 - 42°C.

En fin d'opération d'enrobage, la température des comprimés enrobés avec le film primaire isolant est ramenée à 25°C.

### 2- enrobage avec la matière appétissante

On prépare la dispersion de matière appétissante à pulvériser suivante :
- eau 1966,5 g
- hydroxypropylméthylcellulose / cellulose microcristalline / macrogol stéarate 180,0 g
- Poudre de foie de volaille 459,0 g
pour enrober 2000 g de comprimés rond de 250 mg obtenu à l'étape 1 ci-dessus.

La suspension est au préalable filtrée sur un tamis de 500 µm.

L'enrobage est effectué pendant 5h 20min avec un débit croissant de 2,2 à 11,2 g/min à l'aide d'une buse de 1,5 mm.

La température de l'air entrant est maintenue à environ 50 - 55°C, en vérifiant que la température des comprimés ne dépasse pas 40 - 42°C.

La pulvérisation de la suspension de matière appétissante est arrêtée quand la quantité requise de matière appétissante est atteinte.

En fin d'opération d'enrobage, la température des comprimés enrobés de matière appétissante est ramenée à 25°C.

La quantité de matière appétissante, la poudre de foie de volaille, déposée sur le comprimé est calculée comme suit :
- la masse de 50 comprimés nus est de 12,56 g soit pour un comprimé, 251,2 mg, et
- la masse de 50 comprimés enrobés est de 16,22 g soit pour un comprimé enrobé : 324,4 mg.

Ainsi le poids de l'enrobage est de 73,2 mg constitué de 24,43 mg (33,37% m/m du poids de l'enrobage) de polymère et 48,77 mg (66,63% m/m du poids de l'enrobage) de matière appétissante soit 15,03% p/p de matière appétissante du poids total de comprimé enrobé.

### EXEMPLE 2 - test monadique d'appétence avec les comprimés enrobés de l'exemple 1

Un test d'appétence des compositions appétissantes pharmaceutiques vétérinaires solides à administration orale obtenues dans l'exemple 1 et des mêmes médicaments sans enrobage (comprimés ronds de 250 mg contenant du métronidazole) a été réalisé auprès de trente trois chiens adultes, mâles et femelles, de races variées en test croisé.

Le premier jour, 17 chiens ont testé les compositions appétissantes pharmaceutiques vétérinaires solides à administration orale (comprimés enrobés selon l'exemple 1) et 16 chiens ont testé les comprimés sans enrobage. Le troisième jour, le premier groupe de 17 chiens a testé les comprimés non enrobés tandis que le groupe de 16 chiens a testé les comprimés enrobés selon l'exemple 1.

Il s'agit d'un test monadique réalisé sur deux journées de test et mené dans des box individuels pendant dix minutes par chiens. Ont été mesurés :
- la préhension
   - à la main,
   - au sol, ou
   - pas de prise
- la consommation
   - partielle,
   - totale, ou
   - pas de consommation.

Pour chacun de ces critères est précisé le nombre d'individus, sur l'ensemble du panel et par catégories de taille (petite/moyenne/grande).

Le calcul de l'acceptabilité est basé sur le pourcentage de chiens ayant consommé la totalité du comprimé proposé, enrobé ou non.

### Préhension :

### Comprimés enrobés selon l'invention (exemple 1)

| | petits chiens | chiens moyens | grands chiens |
|---|---|---|---|
| A la main | 11 | 11 | 10 |
| au sol | 0 | 0 | 1 |
| pas de prise | 0 | 0 | 0 |

### Comprimés non enrobés

| | petits chiens | chiens moyens | grands chiens |
|---|---|---|---|
| à la main | 1 | 2 | 0 |
| au sol | 0 | 0 | 2 |
| pas de prise | 10 | 9 | 9 |

### Consommation : prise en compte des seuls animaux ayant pris le produit.

### Comprimés enrobés selon l'invention (exemple 1)

| | petits chiens | chiens moyens | grands chiens |
|---|---|---|---|
| partielle | 0 | 0 | 1 |
| totale | 11 | 11 | 10 |
| pas de consommation | 0 | 0 | 0 |

### Comprimés non enrobés

| | petits chiens | chiens moyens | grands chiens |
|---|---|---|---|
| partielle | 0 | 2 | 0 |
| totale | 1 | 0 | 1 |
| pas de consommation | 0 | 0 | 1 |

L'acceptabilité et la consommation des comprimés enrobés selon l'invention sont totales (97%) alors que celles des comprimés non enrobés ne sont que de 6 %.

### EXEMPLE 3 - Préparation de gélules enrobées selon l'invention

Pour encapsuler 765 g de gélules de 133 mg contenant un mélange de levothyroxine, lactose, acide stéarique, on prépare la dispersion de matière appétissante à pulvériser suivante :
- eau 452 g
- hydroxypropylméthylcellulose / cellulose microcristalline / macrogol stéarate 45 g
- Poudre de foie de volaille 115 g

Les gélules sont introduites dans la turbine à dragéifier où elles sont dépoussiérées et mises en température.

La suspension est au préalable filtrée sur un tamis de 500 µm.

L'encapsulation est effectuée pendant environ 2h10 avec un débit croissant de 3,5 à 4,6 g/min à l'aide d'une buse de 1,8 mm.

La pulvérisation de la suspension de matière appétissante est arrêtée quand la quantité requise de matière appétissante est atteinte.

La température de l'air entrant est maintenue à environ 55°C, en vérifiant que la température des gélules ne dépasse pas 38 - 40°C.

En fin d'opération d'enrobage la température des gélules enrobées est ramenée à 25°C.

La quantité de matière appétissante, la poudre de foie de volaille, déposée sur la gélule est calculée comme suit :
- La masse de 50 gélules non enrobées est de 6,65 g soit pour une gélule, 133 mg, et
- La masse de 50 gélules enrobées est de 8,45 g soit pour une gélule enrobée, 169 mg,

Ainsi le poids de l'enrobage est de 36 mg constitué de 10,08 mg (28% m/m du poids de l'enrobage) de polymère et 25,92 mg (72% m/m du poids de l'enrobage) de matière appétissante soit 15,33% p/p de matière appétissante du poids total de gélule enrobée.

### EXEMPLE 4 - test monadique d'appétence et de stabilité avec les gélules enrobées de l'exemple 3

### a) Appétence

Un test d'appétence des compositions appétissantes pharmaceutiques vétérinaires solides à administration orale obtenues dans l'exemple 3 et du médicament non enrobé (gélules de 133 mg contenant de la levothyroxine ayant un arôme poulet inclus dans la tunique (paroi) de la gélule, représentant moins un pour cent en poids de la gélule vide) a été réalisé auprès de trente cinq chiens adultes, mâles et femelles, de races variées en test croisé.

Le premier jour, 18 chiens ont testé les gélules enrobées de l'exemple 3 et 17 chiens ont testé les gélules non enrobées. Le troisième jour, le premier groupe de 18 chiens a testé les gélules non enrobées tandis que le second groupe de 17 chiens a testé les gélules enrobées de l'exemple 3.

Le test a été réalisé avec le protocole décrit dans l'exemple 2.

Le calcul de l'acceptabilité est basé sur le pourcentage de chiens ayant consommé la totalité du vecteur.

### Préhension :

### Gélules enrobées selon l'invention (exemple 3)

| | petits chiens | chiens moyens | grands chiens |
|---|---|---|---|
| à la main | 12 | 10 | 10 |
| au sol | 0 | 1 | 1 |
| pas de prise | 0 | 0 | 1 |

### Gélules non enrobées

| | petits chiens | chiens moyens | grands chiens |
|---|---|---|---|
| à la main | 9 | 6 | 6 |
| au sol | 2 | 3 | 2 |
| pas de prise | 1 | 2 | 4 |

### Consommation : prise en compte des seuls animaux ayant pris le produit.

### Gélules enrobées selon l'invention (exemple 3)

| | petits chiens | chiens moyens | grands chiens |
|---|---|---|---|
| partielle | 0 | 1 | 0 |
| totale | 12 | 10 | 11 |
| pas de consommation | 0 | 0 | 0 |

### Gélules non enrobées

| | petits chiens | chiens moyens | grands chiens |
|---|---|---|---|
| partielle | 0 | 0 | 2 |
| totale | 8* | 7* | 6* |
| pas de consommation | 3 | 2 | 1 |

| | | | |
|---|---|---|---|
| L'acceptabilité et la consommation totale des gélules enrobées selon l'invention sont de 94%, alors que celles des gélules non enrobées mais aromatisées ne sont au maximum que de 60% de prise et de consommation totale sans prendre en compte la remarque ci-après. * Il y a lieu de mentionner le comportement des animaux avec ce type de support : en général, les chiens ont pris le support en gueule. Compte tenu du pouvoir adhésif de la gélule à la cavité buccale en présence de la salive dû à sa composition en gélatine, les animaux cherchent à recracher la gélule ce qui a entraîné leur percement, qui a eu pour effet de disperser une partie du contenu de cette dernière au sol quand ce n'est pas la gélule qui était rejetée directement d'autant plus que l'animal secouait la tête, gueule vers le bas. Dans ce cas de figure, comme certains animaux ont léché tout ou partie de la composition médicamenteuse au sol, il a été attribué une valeur « consommation totale » à ces occurrences. | | | |

### b) Stabilité

Les compositions appétissantes pharmaceutiques vétérinaires solides à administration orale de l'exemple 3 ainsi que les gélules non enrobées (gélules n°3 de 133 mg contenant de la levothyroxine ayant un arôme poulet inclus dans la tunique (paroi) de la gélule, représentant moins un pour cent en poids de la gélule vide) ont été mis en stabilité à 40°C et 75% d'humidité pendant 3 mois et la teneur en levothyroxine a été déterminée à t₀, t_{1,5mois} et t₃ₘₒᵢₛ par chromatographie liquide haute performance.

| | gélule enrobée selon l'exemple 3 Teneur en levothyroxine % p/p | gélule non enrobée Teneur en levothyroxine% p/p |
|---|---|---|
| t₀ | 100 | 100 |
| t_{1,5mois} | 98,9 | 94,6 |
| t₃ₘₒᵢₛ | 97,8 | 91,0 |

Il apparaît que l'enrobage selon l'invention améliore la stabilité de l'actif.

### EXEMPLE 5 - Préparation de comprimés sécables enrobés selon l'invention

Un lot de comprimés ronds de 450 mg présentant sur une de ses faces deux traits de sécabilité perpendiculaires permettant de fractionner le comprimé en quatre secteurs équivalents et de composition : métronidazole / hydroxypropylcellulose / cellulose microcristalline / glycérides de béhénate / povidone / silice colloïdale / dioxyde de silice colloïdale / acide silicique léger, a été enrobé suivant le procédé décrit dans l'exemple 1.

La quantité de matière appétissante, la poudre de foie de volaille, déposée sur le comprimé est calculée comme suit :
- La masse de 50 comprimés nus est de 22,825 g soit pour un comprimé, 456,5 mg, et
- La masse de 50 comprimés enrobés est de 29,54 g soit pour un comprimé enrobé selon l'invention 590,8 mg,

Ainsi le poids de l'enrobage par comprimé est de 134,3 mg constitué de 44,83 mg (33,38% m/m du poids de l'enrobage) de polymère et 89,47 mg (66,62% m/m du poids de l'enrobage) de matière appétissante soit 15,14% p/p de matière appétissante du poids total de comprimé enrobé.

### EXEMPLE 6 - test d'appétence avec les comprimés sécables enrobés de l'exemple 5

Un test d'appétence des comprimés sécables enrobés contenant du métronidazole obtenus dans l'exemple 5 fractionnés en 4 secteurs a été réalisé auprès de trente quatre chiens adultes, mâles et femelles, de races variées. Chaque animal a reçu ¼ de comprimé enrobé ce qui faisait qu'environ la moitié de la surface de la fraction de comprimé n'était pas enrobée.

Le test a été réalisé avec le protocole décrit dans l'exemple 2.

Le calcul de l'acceptabilité est basé sur le pourcentage de chiens ayant consommé la totalité du quart de comprimé.

### Préhension :

| | petits chiens | chiens moyens | grands chiens |
|---|---|---|---|
| à la main | 12 | 10 | 12 |
| au sol | 0 | 0 | 0 |
| pas de prise | 0 | 0 | 0 |

### Consommation : ne sont pris en compte que les animaux ayant pris le produit.

| | petits chiens | chiens moyens | grands chiens |
|---|---|---|---|
| partielle | 0 | 0 | 0 |
| totale | 12 | 10 | 12 |
| pas de consommation | 0 | 0 | 0 |

L'acceptabilité et la consommation de la composition appétissante pharmaceutique vétérinaire solide à administration orale, même si toute sa surface n'est pas enrobée de matière appétissante, sont totales (100%).

### EXEMPLE 7- Préparation de comprimés selon l'invention

Un lot de comprimés oblongs de 180 mg de composition cephalexine / poudre de foie / povidone réticulée / povidone / cellulose microcristalline / mannitol / stéarate de magnésium a été enrobé suivant le protocole décrit à l'exemple 1.

Les comprimés sont introduits dans la turbine à dragéifier où ils sont dépoussiérés et mis en température.

La quantité de matière appétissante, la poudre de foie de volaille, déposée sur le comprimé est calculée comme suit :
- La masse de 50 comprimés nus est de 9,32 g soit pour un comprimé, 184,6 mg, et
- La masse de 50 comprimés enrobés est de 11,94 g soit pour un comprimé enrobé 238,8 mg,

Ainsi le poids de l'enrobage est de 54,2 mg constitué de 18,09 mg (33,38% m/m du poids de l'enrobage) de polymère et 36,11 mg (66,62% m/m du poids de l'enrobage) de matière appétissante soit 15,12% p/p de matière appétissante du poids total de comprimé enrobé.

### EXEMPLE 8 - test d'appétence des comprimés préparés selon l'exemple 7

Un test d'appétence des comprimés enrobés dans l'exemple 7 et des même comprimés non enrobés (pour rappel, il s'agit de comprimés oblongs de 180 mg contenant de la cephalexine et de la matière appétissante) a été réalisé auprès de vingt six chats adultes, mâles et femelles, de races variées en test croisé.

Le premier jour, 13 chats ont testé les comprimés enrobés selon l'exemple 7 et 13 chats ont testé les comprimés non enrobés. Le troisième jour, le premier groupe de 13 chats a testé les comprimés non enrobés tandis que l'autre groupe de 13 chats a testé les comprimés enrobés selon l'exemple 7.

Le test a été réalisé avec le protocole décrit dans l'exemple 2.

Le calcul de l'acceptabilité est basé sur le pourcentage de chats ayant consommé la totalité du vecteur.

### Préhension :

### Comprimés enrobés (exemple 7)

| | chats |
|---|---|
| à la main | 22 |
| au sol | 3 |
| pas de prise | 1 |

### Comprimés non enrobés

| | chats |
|---|---|
| à la main | 13 |
| au sol | 8 |
| pas de prise | 5 |

### Consommation : ne sont pris en compte que les seuls animaux ayant pris le produit.

### Comprimés enrobés (exemple 7)

| | petits chats |
|---|---|
| partielle | 0 |
| totale | 25 |
| pas de consommation | 0 |

### Comprimés non enrobés

| | chats |
|---|---|
| partielle | 1 |
| totale | 20 |
| pas de consommation | 0 |

L'acceptabilité et la consommation des comprimés enrobés sont totales (96%) alors que celles des mêmes comprimés non enrobés ne sont que de 77%.

## Revendications

1. Utilisation d'une composition d'enrobage apte à être appliquée par un procédé de pelliculage sur une composition pharmaceutique vétérinaire solide à administration orale, **caractérisée** en ce :
(i) qu'elle comprend :
- une matière appétissante pour l'animal cible, se présentant sous forme pulvérulente et consistant en une substance d'origine animale ou végétale directement mise en poudre ;
- au moins un liant choisi parmi les polymères d'alcool polyvinylique, de polyvinylpyrrolidone, les copolymères de vinylpyrrolidone et d'acétate de vinyle, l'acétophthalate de polyvinyle, les celluloses et leurs dérivés, l'acide alginique et ses sels, la zeïne, l'acide hyaluronique, les pectines, la gomme arabique, la gomme adragante, la gomme karaya, la gomme xanthane, les carraghénanes, les polymères pullulan ou agar, le chitosan ou ses dérivés, les carbomères, l'acide acrylique réticulé avec des polyalkényl éthers, les polycarbophiles, les copolymères de méthylvinyl et d'anhydride maléique, les polysaccharides ou le mélange d'au moins deux de ces liants ; étant entendu que lorsque le liant comprend un ou plusieurs polysaccharides, le pourcentage en poids du ou desdits polysaccharides représente 50% ou moins, de préférence 25% ou moins, du poids total de liants ; et
- un solvant choisi parmi l'eau, le méthanol, l'éthanol, l'isopropanol, le propylène glycol, la glycérine ou leur mélange ; et
(ii) qu'elle contient entre 30% et 90% inclus en poids de matière appétissante par rapport au poids total du mélange constitué par le liant et la matière appétissante,
pour l'enrobage par pelliculage d'une composition pharmaceutique vétérinaire solide à administration orale.

2. Utilisation selon la revendication 1, **caractérisée en ce que** ladite matière appétissante pulvérulente est choisie parmi la levure de bière, les farines de viandes, les farines de poissons, les poudres de fromages ou dérivés du lait, la poudre de foie et leur mélange.

3. Utilisation selon la revendication 1 ou 2, **caractérisée en ce que** ladite matière appétissante pulvérulente est constituée de particules ayant un diamètre moyen inférieur à 500 µm, préférentiellement inférieur à 400 µm et plus préférentiellement compris entre 50 et 100 µm.

4. Utilisation selon l'une quelconque des revendications précédentes, **caractérisée en ce qu'**elle comprend en outre au moins un additif choisi parmi les plastifiants ; des stabilisants ; des conservateurs ; des charges ; des surfactants ; des colorants et des porogènes.

5. Utilisation selon l'une quelconque des revendications précédentes, **caractérisée en ce que** ladite composition pharmaceutique vétérinaire solide à administration orale est un médicament pour usage vétérinaire.

6. Utilisation selon l'une quelconque des revendications 1 à 4, **caractérisée en ce que** ladite composition pharmaceutique vétérinaire solide à administration orale est un produit nutraceutique ou un complément nutritionnel destiné aux animaux de compagnies.

7. Utilisation selon l'une quelconque des revendications précédentes, **caractérisée en ce que** ladite composition pharmaceutique vétérinaire solide à administration orale est destinée à un animal carnivore domestique.

8. Procédé de préparation d'une composition pharmaceutique vétérinaire solide à administration orale enrobée par pelliculage d'une matière appétissante, **caractérisé en ce qu'**il comprend les étapes suivantes :
(1) application sur une composition pharmaceutique vétérinaire solide à administration orale d'une couche d'une composition d'enrobage comprenant :
- une matière appétissante pour l'animal cible, se présentant sous forme pulvérulente et consistant en une substance d'origine animale ou végétale directement mise en poudre ;
- au moins un liant choisi parmi les polymères d'alcool polyvinylique, de polyvinylpyrrolidone, les copolymères de vinylpyrrolidone et d'acétate de vinyle, l'acétophthalate de polyvinyle, les celluloses et leurs dérivés, l'acide alginique et ses sels, la zeïne, l'acide hyaluronique, les pectines, la gomme arabique, la gomme adragante, la gomme karaya, la gomme xanthane, les carraghénanes, les polymères pullulan ou agar, le chitosan ou ses dérivés, les carbomères, l'acide acrylique réticulé avec des polyalkényl éthers, les polycarbophiles, les copolymères de méthylvinyl et d'anhydride maléique, les polysaccharides ou le mélange d'au moins deux de ces liants ; étant entendu que lorsque le liant comprend un ou plusieurs polysaccharides, le pourcentage en poids du ou desdits dit polysaccharides représente 50% ou moins, de préférence 25% ou moins, du poids total de liants ; et
- un solvant choisi parmi l'eau, le méthanol, l'éthanol, l'isopropanol, le propylène glycol, la glycérine ou leur mélange ;
ladite composition d'enrobage étant telle qu'elle contient entre 30% et 90% inclus en poids de matière appétissante par rapport au poids total du mélange constitué par le liant et la matière appétissante ;
(2) séchage de ladite composition enrobée obtenue à l'étape (1) ;
(3) optionnellement, répétition des étapes (1) et (2) ; et
(4) obtention d'une composition appétissante pharmaceutique vétérinaire solide à administration orale dans laquelle ladite matière appétissante représente au moins 5% et de préférence au moins 15% en poids de ladite composition appétissante pharmaceutique vétérinaire solide à administration orale.

9. Procédé selon la revendication 8, **caractérisé en ce que** ladite composition pharmaceutique vétérinaire solide à administration orale est un médicament pour usage vétérinaire.

10. Procédé selon la revendication 8 ou la revendication 9, **caractérisé en ce que** ladite composition pharmaceutique vétérinaire solide à administration orale est un comprimé, une gélule, une capsule dure ou molle, une pilule, une tablette, des granulés, un cachet, une gomme à mâcher ou une pastille.

11. Procédé selon la revendication 10, **caractérisé en ce que** ladite composition pharmaceutique vétérinaire solide à administration orale est sécable.

12. Procédé selon l'une quelconque des revendications 8 à 11, **caractérisé en ce qu'**il comprend une étape additionnelle préalable à l'étape (1) consistant à enrober ladite composition pharmaceutique vétérinaire solide à administration orale d'un film primaire à l'aide d'une solution ou suspension comprenant au moins un liant et au moins un solvant et/ou un ou des additifs acceptables.

13. Composition appétissante pharmaceutique vétérinaire solide à administration orale, **caractérisée en ce qu'**elle comprend :
- une composition pharmaceutique vétérinaire solide à administration orale contenant au moins un principe actif ; et
- un enrobage appliqué par pelliculage positionné autour de ladite composition constitué d'une matière appétissante se présentant sous forme pulvérulente consistant en une substance d'origine animale ou végétale directement mise en poudre et d'au moins un liant choisi parmi les polymères d'alcool polyvinylique, de polyvinylpyrrolidone, les copolymères de vinylpyrrolidone et d'acétate de vinyle, l'acétophthalate de polyvinyle, les celluloses et leurs dérivés, l'acide alginique et ses sels, la zeïne, l'acide hyaluronique, les pectines, la gomme arabique, la gomme adragante, la gomme karaya, la gomme xanthane, les carraghénanes, les polymères pullulan ou agar, le chitosan ou ses dérivés, les carbomères, l'acide acrylique réticulé avec des polyalkényl éthers, les polycarbophiles, les copolymères de méthylvinyl et d'anhydride maléique, les polysaccharides, ou le mélange d'au moins deux de ces liants ; étant entendu que lorsque le liant comprend un ou plusieurs polysaccharides, le pourcentage en poids du ou desdits polysaccharides représente 50% ou moins, de préférence 25% ou moins, du poids total de liants ; ledit enrobage contenant entre 30% et 90% inclus en poids de matière appétissante par rapport au poids total de l'enrobage constitué de la matière appétissante et du liant ; ladite matière appétissante représentant au moins 5% et préférentiellement au moins 15% en poids de ladite composition appétissante pharmaceutique vétérinaire solide à administration orale.

14. Composition selon la revendication 13, **caractérisée en ce qu'**elle est sécable.

15. Composition selon la revendication 13 ou la revendication 14, **caractérisée en ce qu'**elle est destinée à un animal carnivore domestique.

## Patentansprüche

1. Verwendung einer Überzugszusammensetzung, die geeignet ist, durch ein Beschichtungsverfahren auf eine feste veterinärpharmazeutische Zusammensetzung zur oralen Verabreichung aufgetragen zu werden, **dadurch gekennzeichnet, dass**
(i) sie umfasst:
- ein Material, das für das Zieltier appetitanregend ist, das in Pulverform vorliegt und aus einer Substanz tierischer oder pflanzlicher Herkunft besteht, die direkt zu Pulver verarbeitet wurde;
- wenigstens ein Bindemittel, ausgewählt aus Polymeren von Polyvinylalkohol, Polyvinylpyrrolidon, Copolymeren von Vinylpyrrolidon und Vinylacetat, Polyvinylacetophthalat, Cellulosen und ihren Derivaten, Alginsäure und ihren Salzen, Zein, Hyaluronsäure, Pektinen, Gummi arabicum, Tragantgummi, Karayagummi, Xanthangummi, Carrageenanen, Pullulan- oder Agarpolymeren, Chitosan oder seinen Derivaten, Carbomeren, Acrylsäure, vernetzt mit Polyalkenylethern, Polycarbophilen, Copolymeren von Methylvinyl und Maleinsäureanhydrid, Polysacchariden oder einem Gemisch von wenigstens zwei dieser Bindemittel; mit der Maßgabe, wenn das Bindemittel ein oder mehrere Polysaccharid(e) umfasst, der Gewichtsprozentanteil des Polysaccharids oder der Polysaccharide 50% oder weniger, vorzugsweise 25% oder weniger des Gesamtgewichts der Bindemittel darstellt, und
- ein Lösungsmittel, ausgewählt aus Wasser, Methanol, Ethanol, Isopropanol, Propylenglycol, Glycerin und deren Gemisch; und
(ii) sie zwischen 30 Gew.-% und 90 Gew.-%, einschließlich, appetitanregendes Material, bezogen auf das Gesamtgewicht des Gemisches, das aus dem Bindemittel und dem appetitanregenden Material besteht, enthält, für den Überzug einer festen veterinärpharmazeutischen Zusammensetzung zur oralen Verabreichung durch Beschichtung.

2. Verwendung gemäß Anspruch 1, **dadurch gekennzeichnet, dass** das pulverförmige appetitanregende Material aus Bierhefe, Fleischmehlen, Fischmehlen, Pulvern von Käsen oder Milchprodukten, Lebermehl und ihrem Gemisch ausgewählt ist.

3. Verwendung gemäß Anspruch 1 oder 2, **dadurch gekennzeichnet, dass** das appetitanregende pulverförmige Material aus Partikeln besteht, die einen mittleren Durchmesser von unter 500 µm, vorzugsweise unter 400 µm und äußerst bevorzugt von zwischen 50 und 100 µm haben.

4. Verwendung gemäß einem der vorangehenden Ansprüche, **dadurch gekennzeichnet, dass** sie außerdem wenigstens ein Additiv umfasst, das aus Weichmachern, Stabilisatoren, Konservierungsmitteln, Füllstoffen, Surfactants, Färbemitteln und porenbildenden Mitteln ausgewählt ist.

5. Verwendung gemäß einem der vorangehenden Ansprüche, **dadurch gekennzeichnet, dass** die feste veterinärpharmazeutische Zusammensetzung zur oralen Verabreichung ein Medikament zur veterinären Verwendung ist.

6. Verwendung gemäß einem der Ansprüche 1 bis 4, **dadurch gekennzeichnet, dass** die feste veterinärpharmazeutische Zusammensetzung zur oralen Verabreichung ein nutrazeutisches Produkt oder ein Nahrungsergänzungsmittel ist, das für Begleittiere bestimmt ist.

7. Verwendung gemäß einem der vorangehenden Ansprüche, **dadurch gekennzeichnet, dass** die feste veterinärpharmazeutische Zusammensetzung zur oralen Verabreichung für ein fleischfressendes Haustier bestimmt ist.

8. Verfahren zur Herstellung einer festen veterinärpharmazeutischen Zusammensetzung zur oralen Verabreichung, die durch Beschichtung mit einem appetitanregenden Material überzogen ist, **dadurch gekennzeichnet, dass** es die folgenden Stufen umfasst:
(1) Auftragung auf eine feste veterinärpharmazeutische Zusammensetzung zur oralen Verabreichung einer Schicht aus einer Überzugszusammensetzung, umfassend:
- ein Material, das für das Zieltier appetitanregend ist, das in Pulverform vorliegt und aus einer Substanz tierischer oder pflanzlicher Herkunft besteht, die direkt zu Pulver verarbeitet wurde;
- wenigstens ein Bindemittel, ausgewählt aus Polymeren von Polyvinylalkohol, Polyvinylpyrrolidon, Copolymeren von Vinylpyrrolidon und Vinylacetat, Polyvinylacetophthalat, Cellulosen und ihren Derivaten, Alginsäure und ihren Salzen, Zein, Hyaluronsäure, Pektinen, Gummi arabicum, Tragantgummi, Karayagummi, Xanthangummi, Carrageenanen, Pullulan- oder Agarpolymeren, Chitosan oder seinen Derivaten, Carbomeren, Acrylsäure, vernetzt mit Polyalkenylethern, Polycarbophilen, Copolymeren von Methylvinyl und Maleinsäureanhydrid, Polysacchariden oder einem Gemisch von wenigstens zwei dieser Bindemittel; mit der Maßgabe, wenn das Bindemittel ein oder mehrere Polysaccharid(e) umfasst, der Gewichtsprozentanteil des Polysaccharids oder der Polysaccharide 50% oder weniger, vorzugsweise 25% oder weniger des Gesamtgewichts der Bindemittel darstellt, und
- ein Lösungsmittel, ausgewählt aus Wasser, Methanol, Ethanol, Isopropanol, Propylenglycol, Glycerin und deren Gemisch;
wobei die Überzugszusammensetzung zwischen 30 Gew.-% und 90 Gew.-%, einschließlich, appetitanregendes Material, bezogen auf das Gesamtgewicht des Gemisches, das aus dem Bindemittel und dem appetitanregenden Material besteht, enthält;
(2) Trocknung der überzogenen Zusammensetzung, die in Stufe (1) erhalten wurde;
(3) gegebenenfalls Wiederholung der Stufen (1) und (2) und
(4) Erhalt einer festen veterinärpharmazeutischen appetitanregenden Zusammensetzung zur oralen Verabreichung, in der das appetitanregende Material wenigstens 5 Gew.-% und vorzugsweise wenigstens 15 Gew.-% der festen veterinärpharmazeutischen appetitanregenden Zusammensetzung zur oralen Verabreichung darstellt.

9. Verfahren gemäß Anspruch 8, **dadurch gekennzeichnet, dass** die feste veterinärpharmazeutische Zusammensetzung zur oralen Verabreichung ein Medikament zur veterinären Verwendung ist.

10. Verfahren gemäß Anspruch 8 oder Anspruch 9, **dadurch gekennzeichnet, dass** die feste veterinärpharmazeutische Zusammensetzung zur oralen Verabreichung eine gepresste Tablette, eine Gelatinekapsel, eine harte oder weiche Kapsel, eine Pille, eine Tablette, ein Granulat, eine Tablette (Cachet), ein Kaugummi oder eine Pastille ist.

11. Verfahren gemäß Anspruch 10, **dadurch gekennzeichnet, dass** die feste veterinärpharmazeutische Zusammensetzung zur oralen Verabreichung teilbar ist.

12. Verfahren gemäß einem der Ansprüche 8 bis 11, **dadurch gekennzeichnet, dass** es eine zusätzliche Stufe vor Stufe (1) umfasst, die darin besteht, die feste veterinärpharmazeutische Zusammensetzung zur oralen Verabreichung mit einem primären Film mittels einer Lösung oder Suspension, die wenigstens ein Bindemittel und wenigstens ein Lösungsmittel und/oder ein annehmbares Additiv oder annehmbare Additive umfasst, zu überziehen.

13. Feste veterinärpharmazeutische appetitanregende Zusammensetzung zur oralen Verabreichung, **dadurch gekennzeichnet, dass** sie umfasst:
- eine feste veterinärpharmazeutische Zusammensetzung zur oralen Verabreichung, die wenigstens einen Wirkstoff enthält, und
- einen Überzug, aufgetragen durch Beschichtung, angeordnet um die Zusammensetzung, bestehend aus einem appetitanregenden Material, das sich in Pulverform präsentiert, bestehend aus einer Substanz tierischer oder pflanzlicher Herkunft, die direkt zu Pulver verarbeitet wurde, und wenigstens einem Bindemittel, ausgewählt aus Polymeren von Polyvinylalkohol, Polyvinylpyrrolidon, Copolymeren von Vinylpyrrolidon und Vinylacetat, Polyvinylacetophtalat, Cellulosen und ihren Derivaten, Alginsäure und ihren Salzen, Zein, Hyaluronsäure, Pectinen, Gummi arabicum, Tragantgummi, Karayagummi, Xanthangummi, Carrageenanen, Pullulan- oder Agarpolymeren, Chitosan und seinen Derivaten, Carbomeren, Acrylsäure, vernetzt mit Polyalkenylethern, Polycarbophilen, Copolymeren von Methylvinyl und Maleinsäureanhydrid, Polysacchariden und einem Gemisch von wenigstens zwei dieser Bindemittel; mit der Maßgabe, dass, wenn das Bindemittel ein Polysaccharid oder mehrere Polysaccharide umfasst, der Gewichtsprozentanteil des Polysaccharids oder der Polysaccharide 50% oder weniger, vorzugsweise 25% oder weniger des Gesamtgewichts der Bindemittel darstellt; wobei der Überzug zwischen 30 Gew.-% und 90 Gew.-%, einschließlich dem appetitanregenden Material, bezogen auf das Gesamtgewichts des Überzugs, das aus dem appetitanregenden Material und dem Bindemittel besteht, enthält; wobei das appetitanregende Material wenigstens 5 Gew.-% und vorzugsweise wenigstens 15 Gew.-% der festen veterinärpharmazeutischen appetitanregenden Zusammensetzung zur oralen Verabreichung darstellt.

14. Zusammensetzung gemäß Anspruch 13, **dadurch gekennzeichnet, dass** sie teilbar ist.

15. Zusammensetzung gemäß Anspruch 13 oder Anspruch 14, **dadurch gekennzeichnet, dass** sie für ein fleischfressendes Haustier bestimmt ist.

## Claims

1. Use of a coating composition capable of being applied by a film-coating process to a solid veterinary pharmaceutical composition for oral administration, **characterized in that**:
(i) it comprises:
- a material appetizing for the target animal, which is provided in the pulverulent form and which is chosen from substances of animal or plant origin, directly brought to a powder after treatment;
- at least one binder chosen from polyvinyl alcohol polymers, polyvinylpyrrolidone polymers, copolymers of vinylpyrrolidone and of vinyl acetate, polyvinyl acetate phthalate, celluloses and their derivatives, alginic acid and its salts, zein, hyaluronic acid, pectins, gum arabic, gum tragacanth, karaya gum, xanthan gum, carrageenans, pullulan or agar polymers, chitosan or its derivatives, carbomers, acrylic acid crosslinked with polyalkenyl ethers, polycarbophils, copolymers of methyl vinyl and of maleic anhydride, polysaccharides or the mixture of at least two of these binders; it being understood that, when the binder comprises one or more polysaccharides, the percentage by weight of said polysaccharides represents 50% or less, preferably 25% or less, of the total weight of binders; and
- a solvent chosen from water, methanol, ethanol, isopropanol, propylene glycol, glycerol or their mixture; and
(ii) it comprises between 30 and 90% inclusive by weight of appetizing material, with respect to the total weight of the mixture composed of the binder and the appetizing material,
in the coating by film-coating of a solid veterinary pharmaceutical composition for oral administration.

2. The use as claimed in claim 1, **characterized in that** said pulverulent appetizing material is chosen from beer yeast, meat meals, fish meals, powdered cheeses or milk derivatives, liver powder and their mixture.

3. The use as claimed in claim 1 or 2, **characterized in that** said pulverulent appetizing material is composed of particles having a mean diameter of less than 500 µm, preferably of less than 400 µm and more preferably of between 50 and 100 µm.

4. The use as claimed in any one of the preceding claims, **characterized in that** it additionally comprises at least one additive chosen from plasticizers; stabilizing agents; preservatives; fillers; surfactants; colorants and porogenic agents.

5. The use of the composition as claimed in any one of the preceding claims, **characterized in that** said solid veterinary pharmaceutical composition for oral administration is a medicament for veterinary use.

6. The use as claimed in claims 1 to 4, **characterized in that** said solid veterinary pharmaceutical composition for oral administration is a nutraceutical product or a nutritional supplement intended for pets.

7. The use as claimed in any one of the preceding claims, **characterized in that** said solid veterinary pharmaceutical composition for oral administration is intended for a domestic carnivorous animal.

8. A process for the preparation of a solid veterinary pharmaceutical composition for oral administration coated with an appetizing material, **characterized in that** it comprises the following stages:
(1) application, to a solid veterinary pharmaceutical composition for oral administration, of a layer of a coating composition comprising:
- a material appetizing for the target animal, which is provided in the pulverulent form and which is chosen from substances of animal or plant origin, directly brought to a powder after treatment;
- at least one binder chosen from polyvinyl alcohol polymers, polyvinylpyrrolidone polymers, copolymers of vinylpyrrolidone and of vinyl acetate, polyvinyl acetate phthalate, celluloses and their derivatives, alginic acid and its salts, zein, hyaluronic acid, pectins, gum arabic, gum tragacanth, karaya gum, xanthan gum, carrageenans, pullulan or agar polymers, chitosan or its derivatives, carbomers, acrylic acid crosslinked with polyalkenyl ethers, polycarbophils, copolymers of methyl vinyl and of maleic anhydride, polysaccharides or the mixture of at least two of these binders; it being understood that, when the binder comprises one or more polysaccharides, the percentage by weight of said polysaccharides represents 50% or less, preferably 25% or less, of the total weight of binders; and
- a solvent chosen from water, methanol, ethanol, isopropanol, propylene glycol, glycerol or their mixture;
said coating composition comprises between 30 and 90% inclusive by weight of appetizing material, with respect to the total weight of the mixture composed of the binder and the appetizing material;
(2) drying said coated composition obtained in stage (1);
(3) optionally, repetition of stages (1) and (2); and
(4) production of a solid veterinary pharmaceutical appetizing composition for oral administration in which said appetizing material represents at least 5% by weight and preferably at least 15% by weight of said solid veterinary pharmaceutical appetizing composition for oral administration.

9. The process as claimed in claim 8, **characterized in that** said solid veterinary pharmaceutical composition for oral administration is a medicament for veterinary use.

10. The process as claimed in any one of claims 8 to 9, **characterized in that** said solid veterinary pharmaceutical composition for oral administration is a tablet, including a compressed tablet, a gelatin capsule, a hard or soft capsule, a pill, a lozenge, granules, a chewing gum or a pastille.

11. The process as claimed in claim 10, **characterized in that** said solid veterinary pharmaceutical composition for oral administration is scored.

12. The process as claimed in any one of claims 8 to 11, **characterized in that** it comprises an additional stage prior to stage (1) which consists in coating said solid veterinary pharmaceutical composition for oral administration with a primer film using a solution or suspension comprising at least one binder and at least one solvent and/or one or more acceptable additives.

13. A solid veterinary pharmaceutical appetizing composition for oral administration, **characterized in that** it comprises:
- a solid veterinary pharmaceutical composition for oral administration comprising at least one active principle; and
- a coating applied by film-coating and positioned around said composition composed of an appetizing material chosen from substances of animal or plant origin, directly brought to a powder after treatment and of at least one binder chosen from polyvinyl alcohol polymers, polyvinylpyrrolidone polymers, copolymers of vinylpyrrolidone and of vinyl acetate, polyvinyl acetate phthalate, celluloses and their derivatives, alginic acid and its salts, zein, hyaluronic acid, pectins, gum arabic, gum tragacanth, karaya gum, xanthan gum, carrageenans, pullulan or agar polymers, chitosan or its derivatives, carbomers, acrylic acid crosslinked with polyalkenyl ethers, polycarbophils, copolymers of methyl vinyl and of maleic anhydride, polysaccharides or the mixture of at least two of these binders; it being understood that, when the binder comprises one or more polysaccharides, the percentage by weight of said polysaccharides represents 50% or less, preferably 25% or less, of the total weight of binders; said coating comprising between 30 and 90% inclusive by weight of appetizing material, with respect to the total weight of the coating composed of the appetizing material and of the binder, said appetizing material representing at least 5% by weight and preferably at least 15% by weight of said solid veterinary pharmaceutical appetizing composition for oral administration.

14. The composition as claimed in claim 13, **characterized in that** it is scored.

15. The composition as claimed in any one of claims 13 to 14, **characterized in that** it is intended for a domestic carnivorous animal.
